Europäisches Patentamt

(19) European Patent Office

Office européen des brevets

(11) Numéro de publication: **0 270 561**
**B1**

(12) **FASCICULE DE BREVET EUROPEEN**

(45) Date de publication du fascicule du brevet: **30.01.91**

(21) Numéro de dépôt: **87902569.0**

(22) Date de dépôt: **12.05.87**

(86) Numéro de dépôt international:
**PCT/FR87/00155**

(87) Numéro de publication internationale:
**WO 87/06834 19.11.87 Gazette 87/25**

(51) Int. Cl.⁵: **A 61 K 35/78**

(54) **MEDICAMENTS DESTINES A LA PREVENTION ET AU TRAITEMENT DE LA TOXICOMANIE ALCOOLIQUE ET LEURS PROCEDES D'OBTENTION.**

(30) Priorité: **12.05.86 FR 8606961**

(43) Date de publication de la demande:
**15.06.88 Bulletin 88/24**

(45) Mention de la délivrance du brevet:
**30.01.91 Bulletin 91/05**

(84) Etats contractants désignés:
**AT BE CH DE FR GB IT LI LU NL SE**

(56) Documents cités:
**US-A-2 567 814**

(73) Titulaire: **FONTAINE, Michel**
**82, rue Joseph Vallot**
**F-74400 Chamonix (FR)**
(73) Titulaire: **BONNEAU, Marc**
**32, rue des Granges**
**F-69005 Lyon (FR)**

(72) Inventeur: **FONTAINE, Michel**
**82, rue Joseph Vallot**
**F-74400 Chamonix (FR)**
Inventeur: **BONNEAU, Marc**
**32, rue des Granges**
**F-69005 Lyon (FR)**

(74) Mandataire: **Maureau, Philippe et al**
**Cabinet Germain & Maureau Le Britannia - Tour**
**C 20, bld Eugène Déruelle Boîte Postale 3011**
**F-69392 Lyon Cédex 03 (FR)**

Courier Press, Leamington Spa, England.

EP 0 270 561 B1

## Description

La présente invention concerne des médicaments nouveaux utilisables en thérapeutique humaine ainsi que leurs procédés d'obtention et leurs caractéristiques physico-chimiques et thérapeutiques.

Ces produits sont destinés à la prévention et au traitement de la toxicomanie alcoolique en raison de leurs activités pharmacologiques capables d'induire chez la plupart des patients traités une désaccoutumance voire un dégoût vis-à-vis des boissons alcoolisées.

L'alcoolisme reste, de nos jours, un fléau médical et social majeur. Par exemple, en France en 1984, la consommation moyenne annuelle d'alcool pur par habitant était de 13,5 litres. Les spécialistes estiment, pour ce pays, le nombre des personnes intoxiquées par l'alcool à cinq millions dont deux millions sont des alcooliques et trois millions sont des buveurs excessifs.

Les retentissements médico-socio-économiques de la toxicomanie alcoolique ont été chiffrés à un coût global d'environ 75 milliards de francs pour la collectivité française pour l'année 1982: frais de maladie liés à l'alcoolisme, accidents du travail, accidents de la circulation, décès prématurés, pertes de journées de travail, etc...

A l'heure actuelle, les traitements proprement dits de désintoxication éthylique mis en oeuvre procèdent de l'utilisation de l'effet antabuse du Disulfirame ainsi que de l'utilisation de la méthode du Docteur Champeau (injection quotidienne intraveineuse de soluté hypertonique à 15% de sulfate de magnésium) qui visent à induire soit une intolérance, soit une désaccoutumance aux boissons alcooliques.

Ces traitements sont difficiles à mettre en oeuvre: risque d'intoxication et d'accidents secondaires dûs au Disulfirame, traitement contraignant pour les injections de sulfate de magnésium et risques d'accidents d'intolérance. Malgré ces inconvénients, ces traitements permettent en moyenne d'obtenir environ 50% de sevrages de bonne qualité chez les malades traités.

Hormis leurs inconvénients médicaux, ces traitements anti-alcooliques imposent des contraintes matérielles, physiques, psychologiques et institutionnelles très lourdes et très coûteuses pour la collectivité et l'économie de la Santé Publique. En outre, ils amènent le malade alcoolique à être totalement pris en charge: sa désintoxication repose sur un principe d'autorité médicale qui tend à se substituer à sa volonté propre et sur un principe de sanction lié aux désagréments induits par l'ingestion d'alcool.

Les inventeurs ont découvert qu'il était possible d'utiliser avec succès, dans les cures anti-alcooliques, de nouveaux médicaments constitués par des extraits utilisés soit isolément, soit en association, et obtenus à partir de deux espèces végétales: le fruit du piment (Capsicum) et le bois et/ou l'écorce de peuplier (Populus).

Les produits selon la présente invention représentent un progrès notable par rapport aux précédentes substances utilisées dans les cures anti-alcooliques par les caractéristiques suivantes:

Ce sont des produits obtenus par extraction et purification à partir de substances végétales. Ils sont totalement dénués de toxicité. Des doses cinquante fois supérieures aux doses thérapeutiques utilisées chez l'homme ont été administrées à des animaux de laboratoire (souris, cobaye) sans induire de manifestations toxiques notables. D'ailleurs, les végétaux desquels sont tirés ces extraits appartiennent à la pharmacopée et sont connus pour leur non-toxicité. Par contre, leur activité anti-alcoolique n'est pas connue à ce jour.

Ce sont des produits administrables par voie orale qui ne nécessitent pas obligatoirement l'hospitalisation du malade. De plus, ces traitements peuvent être mis en oeuvre à l'insu du malade, par sa famille en rajoutant le médicament à ses boissons et à ses aliments habituels. Une prise quotidienne est généralement suffisante.

Ces traitements n'imposent pas de sevrage alcoolique brutal; le patient à désintoxiquer peut avoir accès à ses boissons alcoolisées habituelles dont il réduit lui-même progressivement la consommation au fur et à mesure que les effets thérapeutiques des médicaments objets de l'invention se font sentir.

Ces traitements donnent des résultats qualitativement supérieurs à ceux obtenus par les traitements classiques actuels sus-cités. Les réussites thérapeutiques marquées par un sevrage alcoolique de bonne qualité, d'une durée supérieure à huit mois et dénué de rechute, peuvent être chiffrées à environ 65 à 70% des cas traités.

En conséquence, ces traitements marquent un progrès notable tant par la qualité de leurs résultats que par la baisse des coûts de santé que
que leur mise en oeuvre pourra occasionner comparativement aux traitements existants.

Un procédé de préparation des médicaments, objets de l'invention, constitués soit d'un extrait simple, soit d'un mélange d'extraits végétaux, va maintenant être décrit en détail:

a) PIMENT (genre Capsicum — Variété Capsicum Annuum):

Le premier extrait est obtenu par macération à température ambiante, pendant dix jours, d'un volume de broyat pulvérulent du fruit mûr du piment (genre Capsicum), de préférence la variété piment doux potager (genre Capsicum Annuum) dans dix volumes de solvant constitué d'un mélange eau-alcool éthylique à 60°GL. Le broyat pulvérulent est constitué de particules présentant un diamètre moyen de l'ordre de 200 μm, et peut également provenir des feuilles et tiges de Capsicum Annuum.

Le rapport matières végétales pulvérulentes sur solvant d'extraction peut varier considérablement jusqu'à, par exemple, un volume de broyat de piment potager pour vingt volumes d'alcool éthylique à 60°.

2

Les durées de macération et d'extraction alcooliques peuvent varier, tout en restant efficaces, de quelques heures à plusieurs semaines.

L'extrait alcoolique de Capsicum Annuum ainsi obtenu est ensuite clarifié par centrifugation à 3 000 g pendant 15 mn à 4°C. Le liquide surnageant est recueilli puis filtré sur membranes stérilisantes et réparti en flacon bouché de 10 ml.

Selon l'une des variantes du procédé, l'extrait alcoolique de Capsicum Annuum précédemment obtenu peut être avantageusement fractionné par chromatographie de perméation, puis purifié et concentré par ultrafiltration. Le concentré ainsi obtenu peut également être rendu pulvérulent par lyophilisation, dessication sous vide à basse température.

Les divers paramètres du procédé technique décrit ci-dessus sont fournis à titre d'exemple et ne sont pas limitatifs. Des solvants chimiques autres que l'alcool éthylique peuvent aussi être utilisés tels que l'acide acétique par exemple. De même, les paramètres relatifs aux conditions physico-chimiques de l'extraction peuvent varier: la température, le temps de la réaction, la concentration des réactifs pourront être avantageusement modifiés en fonction d'une extraction par macération, d'une extraction par décoction ou d'une extraction par infusion.

Le genre Capsicum, dans toutes ses variétés sauvages ou potagères, peut être utilisé.

b) PEUPLIER (genre Populus — Variété Populus Nigra):

Le deuxième extrait, objet de l'invention, peut être obtenu selon un procédé d'extraction et de purification-concentration identique à celui décrit ci-dessus pour Capsicum Annuum.

La matière végétale utilisée provient d'un broyage en fines particules de 2 mm de diamètre d'écorce, de bois et de feuilles de Populus (de préférence la variété Populus Nigra ou peuplier noir ou peuplier d'Italie). L'extraction est obtenue par macération dans l'alcool éthylique à 60° pendant dix jours à température ambiante.

Les différents paramètres d'extraction fournis à titre indicatif peuvent varier et être différemment associés de manière avantageuse. Par exemple, une extraction simultanée par macération alcoolique des deux broyats végétaux de piment (genre Capsicum) et de peuplier (genre Populus) peut également constituer une variante satisfaisante du procédé de préparation objet de la demande de brevet.

Les caractéristiques physico-chimiques des extraits de Piment et de Peuplier ainsi obtenus vont maintenant être décrites en référence au dessin schématique annexé, dans lequel:

Figure 1 représente le diagramme d'analyse spectrale d'un extrait de Capsicum Annuum,

Figure 2 est une vue similaire à figure 1 pour l'extrait de Populus Nigra,

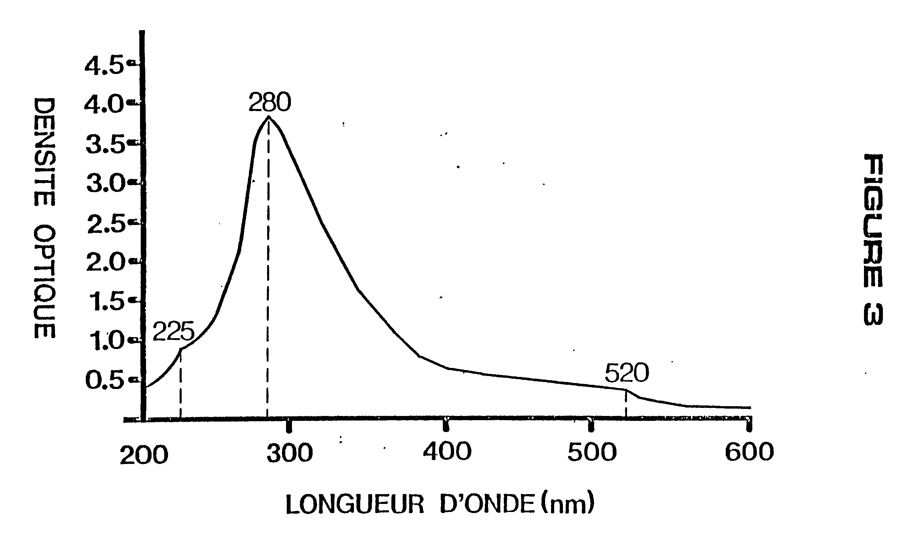

Figure 3 est une vue similaire aux figures 1 et 2 pour le mélange 4/5 Capsicum Annuum, 1/5 Populus Nigra.

a) Extrait de Piment (genre Capsicum):

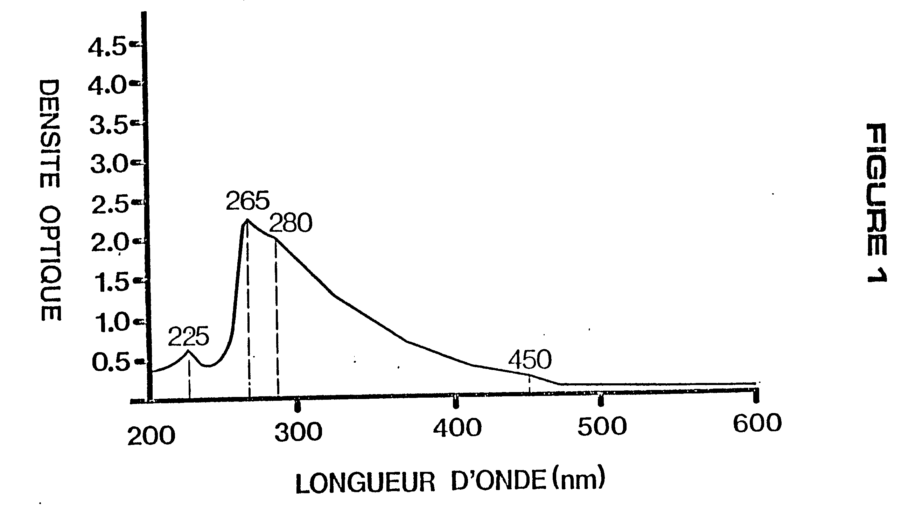

Un millilitre d'extrait de Piment, après évaporation de la phase alcoolique d'extraction, contient 13,9 mg de matières sèches. L'analyse spectrale à diverses longueurs d'onde (cf. figure 1) indique que l'extrait de piment est caractérisé par la présence en quantités diverses de quatre molécules différentes dont les maxima d'absorption caractéristiques respectifs se situent aux alentours de 225 nm, 265 nm, 280 nm et 450 nm.

Les molécules quantitativement les plus abondantes dans l'extrait sont celles caractérisées par les spectres d'absorption maximale respectifs de 265 nm et 280 nm.

L'analyse des poids moléculaires de ces molécules par chromatographie de perméation en tampon phosphate indique que toutes ces molécules présentent des poids moléculaires respectifs égaux ou inférieurs à 300 daltons.

b) Extrait de peuplier (genre Populus)

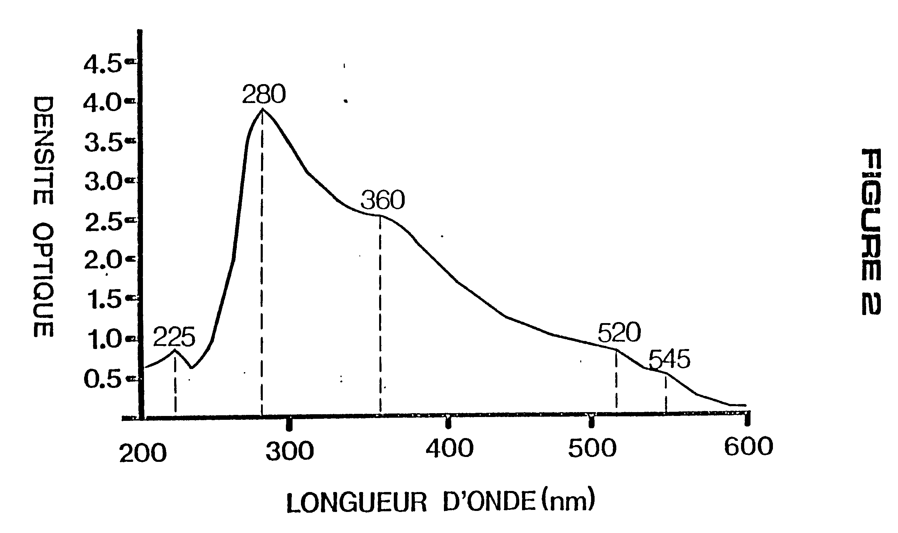

Un millilitre d'extrait de peuplier après évaporation de la phase alcoolique d'extraction contient 19,2 mg de matières sèches. L'analyse spectrale à diverses longueurs d'onde (cf. figure 2) indique que l'extrait de peuplier est caractérisé par la présence en quantités diverses de cinq molécules différentes dont les maxima d'absorption caractéristiques respectifs se situent aux alentours de 225 nm, 280 nm, 360 nm, 520 nm et 545 nm.

Les molécules quantitativement les plus abondantes dans l'extrait sont celles caractérisées par les spectres d'absorption maximale respectifs de 280 nm et 360 nm.

L'analyse des poids moléculaires de ces molécules par chromatographie de perméation en tampon phosphate indique que toutes ces molécules présentent des poids moléculaires respectifs égaux ou inférieurs à 300 daltons.

# EP 0 270 561 B1

| Numéro de l'échantillon | nature de la plante d'origine | poids de 1 ml de solution | poids sec de 1 ml de solution après évaporation |
|---|---|---|---|
| 1 | piment doux (Capsicum Annuum) | 0,8085 g | 13,9 mg/ml |
| 2 | peuplier noir (Populus Nigra) | 0,8540 g | 19,2 mg/ml |
| 3 (cf fig 3) | 4/5 Capsicum Annuum 1/5 Populus Nigra | 0,8078 g | 14,5 mg/ml |

Formes galéniques et posologies

Un des produits selon la présente demande de brevet sera présente à titre indicatif sous deux formes galéniques issues des modes préparatoires sus-cités, soit sous forme de solutions alcooliques utilisant séparément ou en association les extraits alcooliques de Capsicum Annuum (fruit) et/ou de Populus Nigra (écorce et/ou bois), soit sous forme d'extraits secs obtenus par évaporation des solutions précédentes.

Selon les modalités de préparation utilisées dans la présente demande et aux concentrations de produits obtenus correspondent les posologies suivantes citées à titre indicatif:

les solutions alcooliques de Capsicum Annuum, de Populus Nigra ou du mélange Capsicum Annuum (80%) et Populus Nigra (20%) peuvent être utilisées, selon l'état du patient, à des posologies quotidiennes allant de 1 goutte à 50 gouttes par jour, avec un optimum thérapeutique aux alentours de 5 gouttes par jour.

Les extraits secs obtenus par évaporation des solutions alcooliques précédentes sont utilisés à des doses quotidiennes pouvant s'échelonner de 0,3 mg à 15 mg (50 gouttes de solution alcoolique correspondent à 1 ml dont le poids sec résiduel après évaporation est de l'ordre de 13,9 mg à 19,2 mg dans les exemples sus-cités) réparties sous forme soit de gélules, soit de comprimés ou de dragées.

En prenant pour unité de base une posologie quotidienne moyenne de 5 gouttes de solution alcoolique, soit 1,5 mg de résidu sec, les gélules, comprimés ou dragées peuvent être dosés unitairement à cette concentration d'extrait sec. Sous cette forme, la posologie utilisable va de une à dix gélules par jour.

Propriétés pharmacologiques et applications thérapeutiques

Utilisés aux doses quotidiennes sus-citées, les extraits séparés ou associés de Capsicum Annuum (fruit) et de Populus Nigra (écorce et/ou bois), soit sous forme liquide, soit sous forme d'extrait sec, provoquent, lorsqu'ils sont administrés à des patients éthyliques chroniques, une désaccoutumance progressive vis-à-vis des boissons alcoolisées. Soit survient un dégoût vis-à-vis des boissons alcoolisées (10 à 20% des cas positifs), soit une perte progressive du désir et du goût pour ces boissons ainsi que la perte du plaisir et de l'euphorie qu'elles procurent (80 à 90% des cas positifs). Il s'ensuit alors, en cas de résultat positif, un abandon des boissons alcoolisées par les patients traités survenant dans des délais allant de deux à six semaines de traitement. Si au bout d'un mois de traitement effectif, aucune diminution des boissons alcoolisées n'est amorcée par le patient traité, il n'y aura plus à espérer d'effet anti-alcoolique du traitement qui aura alors échoué.

Sur une première série de dix patients ayant reçu le médicament selon l'invention, ont été obtenus huit sevrages alcooliques de longue durée et sans rechute (recul entre deux et quatre ans) contre deux échecs. Une deuxième série plus récente de quatorze patients traités selon les mêmes modalités a confirmé les résultats obtenus précédemment: douze sevrages alcooliques (recul entre six et dix-huit mois) contre deux échecs. Dans ces deux séries, les réussites thérapeutiques ont été objectivées par l'arrêt de la dépendance vis-à-vis des boissons alcoolisées, l'arrêt de leur consommation, la disparition des signes cliniques consécutifs à l'éthylisme chronique et par la normalisation des paramètres biologiques perturbés dans l'éthylisme chronique (gammaglutamyl transpeptidase, transaminases SGOT et SGPT).

En utilisant le produit sous sa forme optimale qui devrait correspondre à un mélange associant 80% d'extrait de Capsicum Annuum (fruit) et 20% d'extrait de Populus Nigra (écorce et/ou bois), on peut s'attendre à obtenir de 60 à 70% de sevrages alcooliques de bonne qualité chez les patients traités.

Le traitement doit être poursuivi pendant une période minimale de deux mois. Il peut être prolongé à la demande et renouvelé si nécessaire. Il garde en principe son activité anti-alcoolique en cas de rechute.

Le traitement, entrepris à l'aide des extraits objets de l'invention, ne provoque ni effets toxiques aigus, ni effets secondaires indésirables. Toutefois, compte tenu du fait que le médicament objet de l'invention contient du piment des jardins ou piment doux, une surveillance médicale doit être mise en oeuvre chez les patients alcooliques ayant présenté ou présentant une pathologie gastro-duodénale du type ulcéreux, afin de déceler d'éventuels signes d'intolérance qui conduiraient à l'arrêt du traitement.

Ce traitement peut être administré au patient à son insu incorporé soit à ses aliments (après leur cuisson; le chauffage au-delà de 60°C détruit le produit), soit mélangé à ses boissons et en particulier ses boissons alcoolisées. Les résultats de désaccoutumance apparaissent sensiblement identiques, que le patient sache ou ne sache pas que ce traitement lui est administré. Un effet thérapeutique de type placebo est donc exclu.

En conséquence, le malade peut avoir accès aux boissons alcoolisées pendant le traitement, ce qui permet d'éviter les mesures autoritaires de sevrage (ces mesures minimisent l'effet psychologique bénéfique et valorisant de l'arrêt de la toxicomanie alcoolique en substituant à la volonté du patient celle de l'équipe médicale). Il en arrive, dans les cas les plus favorables, à se réassumer seul, sans alcool, et sans l'intervention d'une autorité extérieure venant se substituer à son libre choix. Celà lui redonne confiance en lui-même, en sa capacité de volonté, d'autonomie et de succès, ce qui le revalorise à ses yeux et aux yeux de son entourage. Ce point est très important compte tenu du profil psychologique particulier du malade alcoolique. Il efface la vision péjorative que l'alcoolique a de lui-même face à sa démission devant l'alcool et à son incapacité à affronter la réalité sans l'aide de ce toxique; cette vision est en fait un des principaux facteurs responsables de la pulsion d'échec de l'alcoolique qui l'amène à éventuellement rechuter dans sa toxicomanie et à échouer dans sa vie sociale, professionnelle et familiale.

**Revendications pour les Etats contractants: BE CH DE FR GB IT LI LU NL SE**

1. Utilisation d'au moins un extrait d'origine végétale choisi dans le groupe comprenant les extraits des genres Capsicum et Populus, pour l'obtention d'un médicament destiné à la prévention et au traitement de la toxicomanie alcoolique humaine.

2. Utilisation selon la revendication 1, caractérisée en ce qu'on obtient l'extrait de piment (genre Capsicum) par macération, décoction et/ou infusion dans un solvant alcoolo-aqueux.

3. Utilisation selon la revendication 1, caractérisée en ce qu'on obtient l'extrait d'écorce et/ou de bois de peuplier (genre Populus) par macération, décoction, et/ou infusion dans un solvant alcoolo-aqueux.

4. Utilisation selon la revendication 1, caractérisée en ce qu'on utilise un mélange contenant 80% d'extrait du genre Capsicum, et 20% d'extrait du genre Populus.

5. Extrait d'origine végétale du genre Capsicum, pour utilisation comme médicament, caractérisé en ce qu'il contient 13,9 mg de matière sèche spécifique par ml de solvant, constituée d'un mélange de molécules de faible poids moléculaire (PM ≤ 300 D), et qui présentent respectivement un maximum d'absorption spectrale aux longueurs d'onde de 225 nm, 265 nm, 280 nm et 450 nm.

6. Extrait d'origine végétale du genre Populus, pour utilisation comme médicament, caractérisé en ce qu'il contient 19,2 mg de matière sèche spécifique par ml de solvant, constituée d'un mélange de molécules de faible poids moléculaire (PM ≤ 300 D), et qui présentent respectivement un maximum d'absorption spectrale aux longueurs d'onde de 225 nm, 280 nm, 360 nm, 520 nm, 545 nm.

7. Composition d'origine végétale pour utilisation comme médicament, consistant en un mélange contenant 80% d'extrait selon la revendication 5, et 20% d'extrait selon la revendication 6.

8. Extrait ou composition selon l'une quelconque des revendications 5 à 7, caractérisé en ce que l'espèce majoritaire en poids dans la matière sèche présente un spectre d'absorption maximale spécifique à 280 nm.

**Revendications pour l'Etat contractant: AT**

1. Utilisation d'au moins un extrait d'origine végétale choisi dans le groupe comprenant les extraits des genres Capsicum et Populus, pour l'obtention d'un médicament destiné à la prévention et au traitement de la toxicomanie alcoolique humaine.

2. Utilisation selon la revendication 1, caractérisée en ce qu'on obtient l'extrait de piment (genre Capsicum) par macération, décoction et/ou infusion dans un solvant alcoolo-aqueux.

3. Utilisation selon la revendication 1, caractérisée en ce qu'on obtient l'extrait d'écorce et/ou de bois de peuplier (genre Populus) par macération, décoction, et/ou infusion dans un solvant alcoolo-aqueux.

4. Utilisation selon la revendication 1, caractérisée en ce qu'on utilise un mélange contenant 80% d'extrait du genre Capsicum, et 20% d'extrait du genre Populus.

5. Utilisation selon la revendication 1, caractérisée en ce que l'extrait du genre Capsicum contient 13,9 mg de matière sèche spécifique par ml de solvant, constituée d'un mélange de molécules de faible poids moléculaire (PM ≤ 300 D), et qui présentent respectivement un maximum d'absorption spectrale aux longueurs d'onde de 225 nm, 265 nm, 280 nm et 450 nm.

6. Utilisation selon la revendication 1, caractérisée en ce que l'extrait du genre Populus contient 19,2 mg de matière sèche spécifique par ml de solvant, constituée d'un mélange de molécules de faible poids moléculaire (PM ≤ 300 D), et qui présentent respectivement un maximum d'absorption spectrale aux longueurs d'onde de 225 nm, 280 nm, 360 nm, 520 nm, 545 nm.

**Patentansprüche**

1. Verwendung von zumindest einem Auszug pflanzlichen Ursprunges, ausgewählt aus der Gruppe bestehend aus Auszügen der Gattung Capsicum und Populus zum Herstellen eines Arzneimittels zur Vorbeugung und zur Behandlung der menschlichen Alkoholsucht.

2. Verwendung nach Anspruch 1, dadurch gekennzeichnet, daß der Piment-Auszug (Gattung Capsicum) durch Mazeration, Abkochung und/oder Aufgießen mit einem Alkohol-Wasser-Lösungsmittel erhalten wird.

3. Verwendung nach Anspruch 1, dadurch gekennzeichnet, daß der Auszug an Pappelrinde und/oder Pappelholz (Gattung Populus) durch Mazeration, Abkochung und/oder Aufgießen mit einem Alkohol-Wasser-Lösungsmittel erhalten wird.

4. Verwendung nach Anspruch 1, dadurch gekennzeichnet, daß eine Mischung bestehend aus 80% Auszug der Gattung Capsicum und 20% Auszug der Gattung Populus eingesetzt wird.

5. Auszug pflanzlichen Ursprungs der Gattung Capsicum zur Verwendung als Arzneimittel, dadurch gekennzeichnet, daß er 13,9 mg an trockenem spezifischen Material pro ml Lösungsmittel enthält, wobei das Material aus einer Mischung an Molekülen mit geringem Molekulargewicht (MW ≤ 300 D) besteht und die jeweils ein spektrales Absorptionsmaximum bei Wellenlängen von 225 nm, 265 nm, 280 nm und 450 nm aufweist.

6. Auszug pflanzlichen Ursprungs der Gattung populus zur Verwendung als Arneimittel, dadurch gekennzeichnet, daß er 19,2 mg an trockenem spezifischen Material pro ml Lösungsmittel enthält, wobei das Material aus einer Mischung an Molekülen mit geringem Molekulargewicht (MW ≤ 300 D) besteht und die jeweils ein spektrales Absorptionsmaximum bei Wellenlängen von 225 nm, 280 nm, 360 nm, 520 nm und 545 nm aufweist.

7. Zusammensetzung pflanzlichen Ursprunges zur Verwendung als Arzneimittel, bestehend aus einer Mischung aus 80% Auszug nach Anspruch 5 und 20% Auszug nach Anspruch 6.

8. Auszug oder Zusammensetzung nach einem der Ansprüche 5 bis 7, dadurch gekennzeichnet, daß die gewichtsmäßige Mehrheitspezies im trockenem Material ein spezifisches spektrales Absorptionsmaximum bei 280 nm aufweist.

**Claims**

1. Use of at least one extract of vegetable origin, selected from the group comprising extracts of the genera Capsicum and Populus, for obtaining a medicinal product intended for the prevention and treatment of human alcohol addiction.

2. Use according to Claim 1, characterized in that the extract of pepper (genus Capsicum) is obtained by maceration, decoction and/or infusion in an aqueous-alcoholic solvent.

3. Use according to Claim 1, characterized in that the extract of bark and/or of wood of poplar (genus Populus) is obtained by maceration, decoction and/or infusion in an aqueous-alcoholic solvent.

4. Use according to Claim 1, characterized in that a mixture containing 80% of extract of the genus Capsicum and 20% of extract of the genus Populus is used.

5. Extract of vegetable origin of the genus Capsicum, for use as a medicinal product, characterized in that it contains 13.9% of specific dry matter per ml of solvent, consisting of a mixture of molecules of low molecular weight (MW ≤ 300 D) and which have, respectively, a spectral absorption maximum at wavelengths of 225 nm, 265 nm, 280 nm and 450 nm.

6. Extract of vegetable origin of the genus Populus, for use as a medicinal product, characterized in that it contains 19.2 mg of specific dry matter per ml of solvent, consisting of a mixture of molecules of low molecular weight (MW ≤ 300 D) and which have, respectively, a spectral absorption maximum at wavelengths of 225 nm, 280 nm, 360 nm, 520 nm and 545 nm.

7. Composition of vegetable origin for use as a medicinal product, consisting of a mixture containing 80% of extract according to Claim 5 and 20% of extract according to Claim 6.

8. Extract or composition according to any one of Claims 5 to 7, characterized in that the preponderant species, by weight, in the dry matter has a spectrum of specific maximal absorption at 280 nm.

# FIGURE 1

# FIGURE 2

# FiGURE 3